# EUROPEAN PATENT SPECIFICATION

(11) **EP 1 876 231 B1**
(45) Date of publication and mention of the grant of the patent: **17.08.2016**
(21) Application number: 07102369.1
(22) Date of filing: 14.02.2007
(51) Int. Cl.: C12M 1/34

(54) **Microfluidic Device Comprising Microchannel or Microchamber with Hydrophobic Porous Polymer Including Magnetic Beads Bonded to Walls Thereof**
Mikrofluidische Vorrichtung mit einem Mikrokanal oder einer Mikrokammer mit einem hydrophoben porösen Polymer und mit deren Wänden verbundenen magnetischen Kügelchen
Dispositif microfluide comprenant un microcanal ou une microchambre avec un polymère poreux hydrophobique avec des lits magnétiques fixés aux murs correspondants

(30) Priority: 05.07.2006 KR 20060062976
(43) Date of publication of application: 09.01.2008
(73) Proprietor: Samsung Electronics Co., Ltd., Suwon-si, Gyeonggi-do, 443-742 (KR)
(72) Inventor: Yoo, Chang-eun, Gyeonggi-do (KR); Park, Jong-myeon, Gyeonggi-do (KR); Jeong, Sung-young, Gyeonggi-do (KR)
(74) Representative: Grünecker Patent- und Rechtsanwälte PartG mbB

(56) References cited:
- EP-A- 1 650 297
- EP-A- 1 662 008
- WO-A-02/12896
- US-A- 5 834 121
- US-A1- 2004 018 611
- US-B1- 6 632 655
- HOFMANN O ET AL: "LASER BASED DISRUPTION OF BACILLUS SPORES ON A MICROCHIP" SPECIAL PUBLICATION - ROYAL SOCIETY OF CHEMISTRY, ROYAL SOCIETY OF CHEMISTRY, LONDON, GB, 2005, pages 258-260, XP009066976 ISSN: 0260-6291
- PITSILLIDES C M ET AL: "Selective cell targeting with light-absorbing microparticles and nanoparticles" BIOPHYSICAL JOURNAL, NEW YORK, US, US, vol. 84, no. 6, June 2003 (2003-06), pages 4023-4032, XP002428862 ISSN: 0006-3495

<110> Samsung Electronics Co., Ltd.
<120> Microfluidic device comprising microchannel or microchamber with hydrophobic porous polymer including magnetic beads bonded to walls thereof
<130> EP46739FZ106pau
<140> not yet assigned
   <141> herewith
<150> KR 10-2006-0062976
   <151> 2006-07-05
<160> 3
<170> KopatentIn 1.71
<210> 1
   <211> 18
   <212> DNA
   <213> Artificial sequence
<220>
   <223> Forward primer
<400> 1
   tgtatgaaga aggcttcg 18
<210> 2
   <211> 19
   <212> DNA
   <213> Artificial sequence
<220>
   <223> Reverse primer
<400> 2
   aaaggtatta actttactc 19
<210> 3
   <211> 20
   <212> DNA
   <213> Artificial sequence
<220>
   <223> Taqman probe
<400> 3
   gtactttcag cggggaggaa 20

## Description

### BACKGROUND OF THE INVENTION

### 1. Field of the Invention

The present invention relates to a microfluidic device comprising a microchannel or microchamber, wherein a hydrophobic porous polymer is bound to walls of the microchannel or microchamber and magnetic beads are bonded to the hydrophobic porous polymer. Further, the invention relates to a method' of concentrating cells as well as extracting and amplifying DNA using the microfluidic device.

### 2. Description of the Related Art

Microfluidic devices are devices in which at least one inlet and one outlet are connected through a microchannel. A microfluidic device is generally used as a lab-on-a-chip for cell concentration, cell separation, cell disruption and amplification of a nucleic acid.

A magnetic bead is generally an encapsulated metal oxide particle comprising a rigid polymeric coating encapsulating the metal oxide particle. The "metal oxide particle" refers to any oxide of a metal or metal alloy having paramagnetic or super paramagnetic properties. A paramagnetic particle refers to a metal oxide particle, which is susceptible to the application of external magnetic fields, yet is unable to maintain a permanent magnetic domain. The term "rigid" refers to a polymeric coating that is sufficiently cross linked to the extent that the polymeric coating stabilizes the metal oxide particle within the coating so that the particle remains enclosed therein. These magnetic beads can be manufactured using known methods in the field. For example, U.S. Patent Nos. 5,395,688; 5,318,797; and 5,283,079 disclose various magnetic beads.

U.S. Patent No. 5,834,121 discloses a composite magnetic bead comprising
a) a microporous matrix of a first polymer comprising at least one vinyl monomer; and
b) a plurality of primary beads, each primary bead comprising a metal oxide having inducible magnetic properties and a coating of a second polymer that comprises at least one vinyl monomer, said coating encapsulating said metal oxide, wherein said plurality of primary beads is distributed throughout said microporous matrix.

US 2004/018611 A1 discloses a microfluidic device comprising a solid support that is capable of separating magnetic or magnetically-labeled target analytes from non-magnetic materials in a high gradient magnetic separation. The solid support comprises a sample inlet port, a sample outlet port, and at least one microchannel having at least one section with walls comprising magnetic beads and a section devoid of magnetic beads. The magnetic beads may be imbedded in the walls, may be coated onto the inner surface of the walls, or may be packed inside the microchannel.

EP 1 662 008 A2 relates to an apparatus for and a method of purifying nucleic acids of cells or viruses. The apparatus comprises a cell lysis capillary having a sample inlet through which samples, magnetic beads, and a solid support are introduced, a laser generator attached to the capillary, and a magnetic force generator attached to the capillary and fixing the magnetic beads to the capillary wall.

However, a microfluidic device, wherein a matrix of a hydrophobic porous polymer, to which magnetic beads are covalently bound and which polymer is covalently bonded to walls of a microchannel or microchamber, has not been disclosed yet. In this application, the term "bound" and "bonded" are used interchangeably.

In this application, the term "microfluidic device" incorporates the concept of a microfluidic device that comprises microfluidic elements such as, e.g., microfluidic channels (also called microchannels or microscale channels). In this application, the term "microfluidic" refers to a device component, e.g., chamber, channel, reservoir, or the like, that includes at lest one cross-sectional dimension, such as depth, width, length, diameter, etc. of from about 0.1 micrometer to about 1000 micrometer. Thus, the term "microchamber" and "microchannel" refer to a channel and a chamber that includes at lest one cross-sectional dimension, such as depth, width, and diameter of from about 0.1 micrometer to about 1000 micrometer, respectively.

In this application, the term "water contact angle" refers to water contact angle measured by a Kruss Drop Shape Analysis System type DSA 10 Mk2. A droplet of 1.5 µl deionized water is automatically placed on the sample. The droplet was monitored every 0.2 seconds for a period of 10 seconds by a CCD-camera and analyzed by Drop Shape Analysis software (DSA version 1.7, Kruss). The complete profile of the droplet was fitted by the tangent method to a general conic section equation. The angles were determined both at the right and left side. An average value is calculated for each drop and a total of five drops per sample are measured. The average of the five drops is taken the contact angle.

### SUMMARY OF THE INVENTION

The present invention provides a microfluidic device comprising a microchannel or microchamber, wherein a hydrophobic porous polymer is covalently bonded to walls of the microchannel or microchamber and magnetic beads are bonded to the hydrophobic porous polymer, wherein the water contact angle of the hydrophobic porous polymer is from 70° to 90°.

The present invention also provides a method of concentrating cells using the microfluidic device.

The present invention also provides a method of disrupting cells using the microfluidic device.

The present invention also provides a method of amplifying a nucleic acid from the cells disrupted using the microfluidic device.

According to an aspect of the present invention, there is provided a microfluidic device including at least one inlet and at least one outlet that are connected with each other through a microchannel, wherein a hydrophobic porous polymer is bonded to walls of said microchannel or microchamber and magnetic beads are bonded to said polymer, wherein the water contact angle of the hydrophobic porous polymer is from 70° to 90°.

In a preferred embodiment, the microfluidic device further comprises a light-emitting device for providing light inside the microchannel or microchamber.

In another embodiment, the microfluidic device may comprise a PCR reaction means comprising a reaction chamber, which is in fluid communication with the microchannel or the microchamber, and a temperature adjustment means for adjusting the temperature in the reaction chamber, said temperature adjustment means preferably comprising a heater for supplying heat to the reaction chamber, and a cooler for cooling the reaction chamber.

According to another aspect of the present invention, a method of concentrating cells or viruses using the microfluidic device of the present invention comprises the steps of flowing a sample containing cells or viruses through the microchannel or the microchamber of the microfluidic device thereby attaching the cells or viruses to the hydrophobic porous polymer.

According to another aspect of the present invention, there is provided a method of disrupting cells or viruses using the microfluidic device of the present invention, comprising the steps of flowing a sample containing cells or viruses through the microchannel or microchamber of the microfluidic device, thereby attaching the cells or viruses to the hydrophobic porous polymer bonded to the wall of the microchannel or microchamber; and
exposing the hydrophobic porous polymer in the microchannel or the microchamber to light, thereby generating heat disrupting cells or viruses.

According to another aspect of the present invention, there is provided a method of amplifying a target nucleic acid from cells or viruses using the microfluidic device of the present invention,comprising the steps of flowing a sample containing the cells or viruses through the microchannel or microchamber of the microfluidic device, thereby attaching the cells or viruses to the hydrophobic porous polymer bonded to the wall of the microchannel or microchamber; exposing the hydrophobic porous polymer in the microchannel or the microchamber to light, thereby generating heat disrupting cells or viruses; and carrying out PCR using the lysates of the disrupted cells or viruses as a template.

### BRIEF DESCRIPTION OF THE DRAWINGS

The above and other features and advantages of the present invention will become more apparent by describing in detail exemplary embodiments thereof with reference to the attached drawings in which:
FIG. 1 includes SEM images of a polybutylmethacrylate to which magnetic beads are bonded, and which is synthesized in a microchamber according to an embodiment of the present invention.
FIG. 2 is a view showing an electrophoresis result of a PCR product, wherein E. *coli* cells were concentrated within a microchamber, to which a hydrophobic porous polymer is covalently bonded and magnetic beads are bonded to said hydrophobic porous polymer; the *E*. *coli* cells were disrupted using a laser, and PCR was carried out using the cell lysates directly as the PCR template.

### DETAILED DESCRIPTION OF THE INVENTION

Hereinafter, the present invention will be described in more detail with reference to the accompanying drawings, in which exemplary embodiments of the invention are shown.

The present invention provides a microfluidic device including at least one inlet and at least one outlet that are connected through a microchannel, wherein a hydrophobic porous polymer is bonded to a wall of the microchannel or microchamber and magnetic beads are bonded to the hydrophobic porous polymer.

In the present invention, a "microfluidic device" is a device wherein at least one inlet and one outlet are connected with each other through a microchannel. Therefore, any device wherein an inlet and an outlet are connected through a microchannel is a microfluidic device regardless of the name thereof. For example a microchip is a type of microfluidic device. A microfluidic device is generally used as a lab-on-a-chip for concentrating cells, separating cells, disrupting cells and amplifying a nucleic acid. However, the microfluidic device according to the present invention is not necessarily used as a lab-on-a-chip for cell concentrating, cell separating, disrupting cells and amplifying a nucleic acid, but can be a device simply including an inlet and an outlet, wherein the inlet and outlet are connected through a microchannel or including a microchamber connected through a microchannel.

The hydrophobic porous polymer according to the present invention is a polymer having a water contact angle from 70° to 90°, which can concentrate materials such as cells or viruses by attaching the cells or viruses thereto. Examples of the hydrophobic porous polymer include a polymer, a copolymer, a cross-linked polymer, etc. and may be cross-linked to form a network matrix.

In the microfluidic device according to the present invention, the hydrophobic porous polymer can be a polymer or a copolymer composed of a vinyl monomer, which has a vinyl double bond and which can be polymerized through a free radical polymerization reaction. Therefore, the vinyl monomer can include a monomer having an acryl group. The vinyl monomer can be a monomer selected from the group consisting of a C1-C20 alkyl acrylate, a C1-C20 alkyl methacrylate and a styrene, but is not limited thereto.

In the microfluidic device according to the present invention, the hydrophobic porous polymer can be a cross-linked polymer composed of a vinyl monomer and a cross-linkable monomer. The cross-linkable monomer is not limited. For example, an aliphatic cross-linkable monomer or an aromatic cross-linkable monomer can be used. The aliphatic cross-linkable monomer can be a monomer selected from the group consisting of an ethyleneglycol dimethacrylate, an ethyleneglycol diacrylate, a tri-methylol propane diacrylate, a tri-methylol propane triacrylate, a tri-methylol propane dimethacrylate, a tri-methylol propane trimethacrylate, a divinylketone, an arylacrylate, a diallyl maleate, a diallyl fumarate, a diallyl succinate, a diallyl carbonate, a diallyl malonate, a diallyl oxalate, a diallyl adipate, a diallyl sebacate, a divinyl sebacate, a N,N'-methylenediacrylamide and a N,N'-methylenedimethacrylamide. Also, the aromatic cross-linkable monomer can include a monomer selected from the group consisting of a divinylbenzene, a trivinylbenzene, a divinyltoluene, a divinylnaphthalene, a diallylphthalate, a divinylxylene and a divinylethylbenzene.

In the microfluidic device according to the present invention, the magnetic beads can be covalently bonded to the hydrophobic porous polymer. The covalent bond between the hydrophobic porous polymer and the magnetic beads can be formed using any known method in the field, for example, by functionalizing the surface of the magnetic beads to have one or more vinyl double bonds and carrying out an ion radical polymerization in the presence of the vinyl monomer and/or the cross-linkable monomer. In addition, the bond between the hydrophobic porous polymer and the wall of the microchannel or microchamber may be a covalent bond, which can be formed using a conventional method. For example, the covalent bond can be formed by synthesizing the hydrophobic porous polymer, to which the magnetic beads are bonded, and reacting the hydrophobic porous polymer to a functionalized wall. The wall can be functionalized to have a vinyl functional group, which can be free radical polymerized, and then carrying out the free radical reaction between the polymer and the wall, optionally in the presence of functionalized magnetic beads, in the microchannel or microchamber.

The magnetic beads used in the present invention can be any conventional magnetic beads. Examples of the magnetic beads that can be used in the present invention are disclosed in U.S. Patent Nos. 5,395,688; 5,318,797; and 5,283,079, each disclosure of which is incorporated herein by reference.

In the microfluidic device according to one embodiment of the present invention, the hydrophobic porous polymer is the product obtained from a photopolymerization reaction of a vinyl monomer, a magnetic bead having a vinyl group on the surface thereof, a cross-linkable monomer having two or more free radical polymerizable double bonds, a photopolymerization initiator and a functional group having a free radical polymerizable vinyl double bond, which functional group is present on the wall of the microchannel or microchamber.

In the microfluidic device according to one embodiment of the present invention, the photopolymerization reaction is carried out by injecting 15 to 33 parts by weight of the magnetic beads, 25 to 75 parts by weight of the cross-linkable monomer, and 0.5 to 1.3 parts by weight of the photopolymerization initiator based on 100 parts by weight of the vinyl monomer into the microchannel or microchamber, and exposing the same to UV radiation.

According to the current embodiment of the present invention, the vinyl monomer can be a monomer selected from the group consisting of a C₁-C₂₀ alkyl acrylate, a C₁-C₂₀ alkyl methacrylate and a styrene. Also, the cross-linkable monomer can be a monomer selected from the group consisting of an ethylene glycol dimethacrylate, an ethylene glycol diacrylate, a tri-methylol propane diacrylate, a tri-methylol propane triacrylate, a tri-methylol propane dimethacrylate, a tri-methylol propane trimethacrylate, a divinylketone, an arylacrylate, a diallyl maleate, a diallyl fumarate, a diallyl succinate, a diallyl carbonate, a diallyl malonate, a diallyl oxalate, a diallyl adipate, a diallyl sebacate, a divinyl sebacate, a N,N'-methylenediacrylamide, and a N, N'-methylenedimethacrylamide, and may be a tri-methylol propane trimethacrylate.

According to the current embodiment of the present invention, the photopolymerization initiator can be a 2,2-dimethoxy-2-phenylacetophenone.

The microfluidic device according to the present invention may further include a light-emitting device for providing light inside the microchannel or microchamber. Any light-emitting device suitable for emitting light in a wavelength range that can be absorbed by the magnetic beads can be used. For example, the light-emitting device can be a laser by means of which a laser beam can be applied onto the magnetic beads, which are bonded to the hydrophobic porous polymer within the microchannel or microchamber. The light-emitting device, for example, a laser, can be located inside or outside the microchannel or microchamber. When the light-emitting device is located outside the microchannel or microchamber, it can be attached to or be detached from the microchannel or microchamber, and at least one wall of the microchannel or microchamber is made from a light-transparent material. The light-emitting device, for example, a laser, can apply a laser beam onto the magnetic beads to induce laser heat and disrupt cells in a sample and denature biological material such as a protein.

The microfluidic device according to the present invention can further comprise a Polymerase Chain Reaction (PCR) reaction means including a reaction chamber, which is in fluid communication with the microchannel or microchamber, and a temperature adjustment means for adjusting the temperature in the reaction chamber. The temperature adjustment means may comprise a heater, which supplies heat to the reaction chamber, and a cooler, which dissipates the heat, i.e. cools the reaction chamber. Therefore, the microfluidic device according to the current embodiment of the present invention can concentrate and disrupt biological samples containing cells in the microchannel or microchamber, and carry out PCR directly using the obtained cell lysates.

A method of concentrating cells or viruses within the microchannel or microchamber according to the present invention includes flowing a sample containing the cells or viruses through the microchannel or microchamber of the microfluidic device according to the present invention, thereby attaching cells or viruses to the hydrophobic porous polymer.

Since the hydrophobic porous polymer is bonded to the microchannel or microchamber, cells or viruses attach to the hydrophobic porous polymer. The sample containing cells or viruses may have a pH value from pH 2.0 to pH 7.0, and preferably from pH 2.5 to pH 4.0.

According to another embodiment of the present invention, there is provided a method of disrupting cells or viruses, including the steps of flowing a sample containing cells or viruses through the microchannel or microchamber of the microfluidic device according to the present invention, thereby attaching cells or viruses to the hydrophobic porous polymer. The microfluidic device for performing the method of disrupting cells or viruses may further comprises a light-emitting device for providing light inside the microchannel or microchamber in order to expose the hydrophobic porous polymer in the microchannel or the microchamber to light thereby generating heat disrupting cells or viruses.

The light according to the current embodiment of the present invention is light having a wavelength or a wavelength range, which can be absorbed by the magnetic beads. The light-emitting device can be any light-emitting device suitable for emitting light in the wavelength range, which the magnetic beads can absorb. An example of the light-emitting device can be a laser generator.

The method of disrupting cells or viruses according to the present invention includes flowing samples containing cells or viruses through the microchannel or microchamber of the microfluidic device according to the present invention, thereby attaching cells or viruses to the hydrophobic porous polymer.

The method of disrupting cells or viruses according to the current embodiment of the present invention further includes disrupting cells or viruses, which are attached to the polymer, by the heat generated upon exposing the hydrophobic porous polymer, in particular the magnetic beads incorporated in said polymer, in the microchannel or the microchamber to light. Lights such as laser light is applied to the hydrophobic porous polymer and absorbed by with the magnetic beads of the polymer and converted to heat, thereby disrupting the cells or viruses.

According to another embodiment of the present invention, there is provided a method of amplifying a target nucleic acid from cells or viruses, the method including: flowing a sample containing cells or viruses through the microchannel or microchamber of the microfluidic device according to the present invention, thereby attaching cells or viruses to the hydrophobic porous polymer. In the embodiment, the microfluidic device may further comprise a light-emitting device for providing light inside the microchannel or microchamber, and a PCR reaction means including a reaction chamber, which is in fluid communication with the microchannel or the microchamber, and a temperature adjustment means, preferably comprising a heater, which supplies heat to the reaction chamber and a cooler, which dissipates the heat and cools the reaction chamber. By means of the light-emitting means, the hydrophobic porous polymer in the microchannel or the microchamber can be exposed to light thereby generating heat disrupting cells or viruses. By means of the PCR reaction means, PCR can be carried out using the lysates of the disrupted cells or viruses as a template.

The light according to the current embodiment of the present invention has a wavelength or wavelength range, which the magnetic beads can absorb, and the light-emitting device can be any light-emitting device emitting light in the wavelength range, which the magnetic beads can absorb. An example of the light-emitting device can be a laser generator.

The method of amplifying a target nucleic acid from cells or viruses according to the current embodiment of the present invention includes: flowing a sample containing cells or viruses through the microchannel or microchamber of the microfluidic device according to the present invention, thereby attaching cells or viruses to the hydrophobic porous polymer and exposing the hydrophobic porous polymer in the microchannel and the microchamber to light resulting in the generation of heat disrupting cells or viruses. Each of these processes is described above.

The method of amplifying the target nucleic acid from cells or viruses further includes the step of carrying out a PCR using the lysates of the disrupted cells or viruses as a template. A Polymerase Chain Reaction (PCR) is well known in the field, and is disclosed in detail in U.S. Patent Nos. 4,683,195; 4,683.202; and 4,965,188, each disclosure of which is incorporated herein by reference. PCR relates to amplifying a target DNA, which is also referred to as a "template", *in vitro* in the presence of primers, which determine the DNA template by hybridizing to the beginning and the end of the template, a polymerase, such as the DNA polymerase, and dNTPs under appropriate conditions. During PCR, the amount of DNA having the same sequence as the target DNA exponentially increases by carrying out a series of cycles comprising the steps of denaturizing the dsDNA, annealing the primers to the denatured ssDNAs, and extension or elongation, i.e. copying the DNA by DNA polymerase starting at the annealed primers, as many times as desired.

The PCR in the method of amplifying the target nucleic acid from the cells or viruses is carried out in the PCR reaction means within the microfluidic device.

Hereinafter, the present invention will be described in more detail with reference to the following examples. However, the following examples are provided for illustrative purposes only and thus the scope of the present invention is not limited thereto or thereby.

### Examples

### Example 1: Synthesis of a hydrophobic porous polymer which is copolymerized with magnetic beads in a microchannel or a microchamber

Magnetic beads and the surface of a microchamber with a volume of 6 µl of a microchip for TMC-1000 (Samsung Techwin) were each individually functionalized by reacting the same with 3-(trimethoxysilyl)propyl methacrylate, to form a vinyl functional group. The microchamber of the microchip was manufactured by bonding wet-etched silicon and glass having pores. Then, the functionalized magnetic beads, butylmethacrylate as a vinyl monomer, trimethylolpropane trimethacrylate as a cross-linkable monomer, and 2,2-dimethoxy-2-phenylacetophenone as a photopolymerization initiator were photopolymerized by irradiating UV in the functionalized microchannel to manufacture a microchannel, wherein a polymerized polybutylmethacrylate containing magnetic beads was covalently bonded to the walls of the microchannel or microchamber.

### (1) Methacrylate functionalization of magnetic beads

1ml (40mg) of magnetic beads (BIOCINE Corporation, radius of 1 µm) having a silicon oxide surface was placed in a microtube and washed three times with 0.1 N NaOH, once with distilled water, three times with 0.1 N HCl, once with distilled water, and three times with acetone. The magnetic beads were separated from the solution using a magnet and reacted with 30 % 3-(trimethoxysllyl)propyl methacrylate solution (v/v, acetone) by mixing for 12 hours. After the reaction ended, the reaction solution was washed three times with acetone, three times with methanol, suspended and stored in 1 ml of methanol.

### (2) Methacrylate functionalization of inner surface of the microchamber

0.1 N NaOH was added to a microchamber (volume 6 µl) of a microchip for TMC-1000 (Samsung Techwin) and left for 20 minutes in the chamber. Then, the solution was removed from the microchamber and the microchamber was washed with water. 0.1 N HCl was added to the microchamber and left for 20 minutes in the chamber. Then, the solution was removed from the microchamber and the microchamber was washed with water and acetone, respectively.

After the microchamber was dried, 30 % 3-(trimethoxysilyl)propyl methacrylate solution (v/v, acetone) was added thereto, left in the chamber at room temperature for 12 hours, washed three times with acetone, and dried in an oven.

### (3) Synthesis of butylmethacrylate copolymer

A solution containing 15 weight-% of butylmethacrylate, 10 weight-% of trimethylolpropane trimethacrylate (TRIM), 50 weight-% of methanol, and 25 weight-% of hexane was manufactured, and 10 mg of 2,2-dimethoxy-2-phenylacetophenone was dissolved therein.

To the above solution, magnetic beads, which were obtained by removing 100 µl of methacrylate functionalized magnetic beads obtained in (1) using a magnet, were added and mixed. This solution was injected into the microchamber, which was methacrylate functionalized as described in (2), and exposed to light having a wavelength of 254 nm at energy of 5000 mJ/cm² for 4 minutes using a UV-crosslinker (CL-1000). The solution of the reactants was removed by centrifuging the chip itself, and the reaction product was washed three times with methanol and dried in an oven. As a control group, a polymer to which magnetic beads were not bound was synthesized in the same manner as used above except the process of mixing magnetic beads.

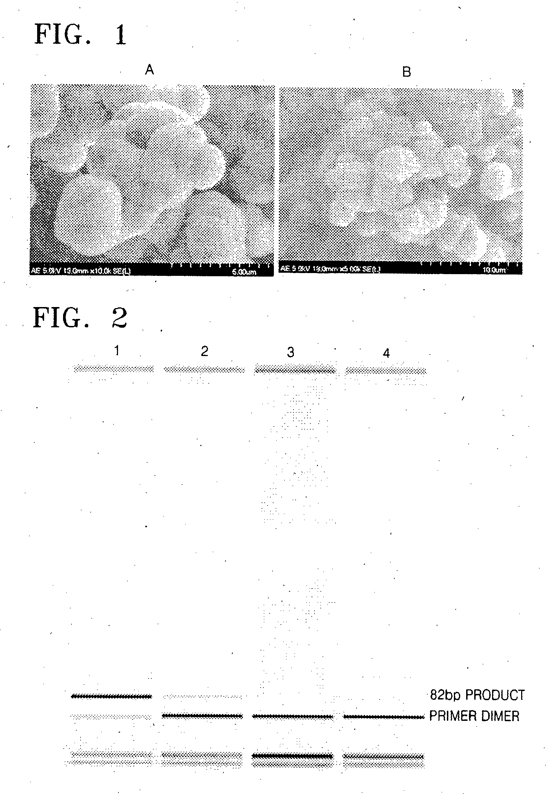

FIG. 1 is a view showing a polymerized polybutylmethacrylate synthesized in a microchamber, to which magnetic beads are bound. As shown in FIG. 1, many pores exist in a polymerized polybutylmethacrylate.

### Example 2: Cell concentrating in a microchamber to which a hydrophobic porous polymer comprising magnetic beads was bonded,

Cells were concentrated by flowing a sample containing cells into a microchamber to the wall of which microchamber a polymerized polybutylmethacrylate polymer comprising magnetic beads was bonded. First, *E*. *coli* was added to 100 mM phosphate buffer (pH 4.0) to make a 0.01 OD₆₀₀ solution. The *E. coli* solution was flowed through a microchamber, prepared according to Example 1, at a rate of 50 µl/min for 3 minutes. A sample of the *E*. *coli* solution, before and after flowing the *E. coli* solution through the microchamber, was diluted 10,000 times with a 1x PBS, and smeared on 3M PetriFilm, which is used for cell culture. Then, the cell concentrations before and after flowing the *E. coli* solution through the microchamber were measured by counting the number of grown colony of *E. coli.* Based on this measurement, the binding ratio, that is, the ratio of *E*. *coli* that were bound to polymers in the microchamber with regard to the *E.coli* cells in the initial solution that entered the microchannel was calculated by comparing the number of cells before and after flowing through the microchamber and then a concentration ratio was calculated by comparing the volume of solution flowing through the microchamber and the volume of the microchamber. The results are shown in Table 1.

**Table 1**

| | **Polymer-magnetic bead** | **Polymer** |
|---|---|---|
| Concentration ratio (%)* | 13.0 (±2.7). | 14.3 (±0.9) |

| | | |
|---|---|---|
| *where concentration ratio: binding ratio x input volume / microchamber volume. | | |

As shown in Table 1, *E. coli* was concentrated more than 10 times whether or not magnetic beads were bound to the polymer.

### Example 3: Cell concentration, cell disruption and PCR using the cell lysates in a microchamber to which a hydrophobic porous polymer containing magnetic beads was bound

The *E*. *coli,* which were concentrated in Example 2, were disrupted in the microchamber using a laser, and the lysates were directly used for PCR to amplify DNA of *E*. *coli.*

First, *E. coli* was concentrated as described in Example 2. The solution was removed from the microchamber, and Tris (10 mM, pH 9.0) or 1x PBS solution was filled in the chamber. Then, the polymer-magnetic bead was exposed to laser light having a wavelength of 808 nm, which was irridiated through the top substrate of the microchamber, using a laser generator (Hamamatsu 8446-72 Corporation, 1W) for 40 seconds. The resulting cell lysates were directly used for PCR.

A PCR master mixture comprising 20 µl of 10x Solgent buffer, 4 µl of Tag polymerase (Solgent), 4 µl of dNTP (10 mM, Solgent), 10 µl of primer (10 µM) (forward and reverse primer having SEQ ID Nos: 1 and 2, respectively) and 8 µl of TaqMan probe (10 µM) (SEQ ID No: 3) (total 56 µl) was mixed with the cell lysate removed from the microchamber in the ratio of 3:1 (v/v), and 1 µl of the resulting mixture was injected into a reaction chamber of TMC-1000, and PCR was carried out.

The PCR was carried out at 95 °C for 1 minute for denaturation, followed by 40 cycles comprising the temperate profile of 5 seconds at 95 °C, 20 seconds at 45 °C and 20 seconds at 72 °C. Ct, indicating after how many PCR-cyles a predetermined threshold value of detection was reached, was measured concurrently carrying out real-time PCR. The results are shown in Table 2.

**Table 2**

| **Elution solution** | **Ct Polymer-magnetic bead** | **Ct Polymer** | **Ct before concentration** |
|---|---|---|---|
| PBS | 26.81 | 31.23 | 30.19 |
| Tris | 22.50 | 25.90 | - |

As shown in Table 2, the value Ct decreased when a polymer-magnetic bead was used compared to a polymer without magnetic beads. The reason for this is believed to be because the cell disruption by a laser light is more efficient when magnetic beads are present. Ct value represents the number of cycles at which detectable fluorescence signals are detected in a real-time PCR. That is, the higher an initial concentration of DNA is, the more fluorescence signals can be detected at a low Ct value.

The PCR product was subjected to an electrophoresis, and the resulting product was analyzed using a LabChip (Agilent Corporation). The results of the analysis are shown in Table 3.

**Table 3**

| **Lane** | **Polymer type** | **buffer** | **DNA concentration (ng/µl)** |
|---|---|---|---|
| 1 | Polymer-magnetic bead | Tris | 8.1 |
| 2 | Polymer only | Tris | 1.4 |
| 3 | Polymer-magnetic bead | PBS | <0.5 |
| 4 | Polymer only | PBS | <0.5 |

As shown in Table 3, the product was much more amplified in the copolymer with magnetic beads when Tris buffer was used as a disrupting buffer, which is consistent with the Ct value given in Table 2. On the other hand, the PCR product was rarely produced and only primer dimer existed when PBS buffer was used as a disrupting buffer, which is inconsistent with the Ct value. The reason for this may be that DNA attached to the surface of the polymer after cell disruption.

FIG. 2 is a view showing an electrophoresis result of a product of PCR, wherein *E. coli* cells were concentrated within a microchamber, to which a hydrophobic porous polymer is covalently bonded, wherein magnetic beads are bound to said hydrophobic porous polymer; the *E. coli* cells were disrupted using a laser; and PCR was carried out directly using the cell lysates as a template. Explanation of each lane is shown in Table 3 above.

At least one process of concentrating cells, disrupting cells, and amplifying a nucleic acid from the cells can be carried out efficiently using the microfluidic device according to the present invention.

Cells or viruses can be efficiently concentrated in the microchannel or microchamber using the method of concentrating cells or viruses in the microchannel or microchamber according to the present invention.

Cells or viruses can be efficiently disrupted in the microchannel or microchamber using the method of disrupting cells or viruses according to the present invention.

A target nucleic acid can be efficiently amplified using the method of amplifying a target nucleic acid from cells or viruses according to the present invention.

While the present invention has been particularly shown and described with reference to exemplary embodiments thereof, it will be understood by those of ordinary skill in the art that various changes in form and details may be made therein without departing from the spirit and scope of the present invention as defined by the following claims.
## Claims

1. A microfluidic device comprising at least one inlet and at least one outlet that are connected with each other through a microchannel or microchamber, wherein a hydrophobic porous polymer is bound to the wall of the microchannel or microchamber and magnetic beads are bound to the hydrophobic porous polymer, wherein the water contact angle of the hydrophobic porous polymer is from 70° to 90°

2. The device of claim 1, further comprising a light-emitting device for providing light inside the microchannel or the microchamber.

3. The device of claim 1 or 2, further comprising a PCR reaction means comprising:
a reaction chamber, which is in fluid communication with the microchannel or the microchamber; and
a temperature adjustment means for adjusting the temperature in the reaction chamber.

4. The device of claim 3, wherein the temperature adjustment means comprises a heater for providing heat to the reaction chamber and a cooler for cooling the reaction chamber.

5. The device of any of claims 1 to 4, wherein the hydrophobic porous polymer is a polymer of vinyl monomers.

6. The device of claim 5, wherein each of the vinyl monomers is a monomer selected from the group consisting of a C₁-C₂₀ alkyl acrylate, a C₁-C₂₀ alkyl methacrylate, and a styrene.

7. The device of any of claims 1 to 6, wherein the hydrophobic porous polymer is a cross-linked hydrophobic porous polymer.

8. The device of claim 7, wherein the hydrophobic porous polymer is cross-linked by an aliphatic cross-linkable monomer.

9. The device of claim 8, wherein the aliphatic cross-linkable monomer is a monomer selected from the group consisting of an ethylene glycol dimethacrylate, an ethylene glycol diacrylate, a tri-methylol propane diacrylate, a tri-methylol propane triacrylate, a tri-methylol propane dimethacrylate, a tri-methylol propane trimethacrylate, a divinylketone, arylacrylate, diallyl maleate, diallyl fumarate, diallyl succinate, diallyl carbonate, diallyl malonate, diallyl oxalate, diallyl adipate, diallyl sebacate, divinyl sebacate, N,N'-methylene diacrylamide, and N,N'-methylene dimethacrylamide.

10. The device of any of claims 1 to 9, wherein the hydrophobic porous polymer is cross-linked by an aromatic cross-linkable monomer.

11. The device of claim 10, wherein the aromatic cross-linkable monomer is a monomer selected from the group consisting of a divinylbenzene, a trivinylbenzene, a divinyltoluene, a divinylnaphthalene, a diallylphthalate, a divinylxylene, and a divinylethylbenzene.

12. The device of any of claims 1 to 11, wherein the magnetic beads are covalently bound to the hydrophobic porous polymer.

13. The device of any of claims 1 to 12, wherein the hydrophobic porous polymer is a product which is obtainable from a photopolymerization of a vinyl monomer, magnetic beads having a vinyl group at the surface thereof, a cross-linkable monomer having two or more free radical polymerizable double bonds, a photopolymerization initiator, and a functional group having a free radical polymerizable vinyl double-bond, said functional group existing in the wall of the microchannel or microchamber.

14. The device of claim 13, wherein the photopolymerization is carried out by:
injecting 15 to 33 parts by weight of the magnetic beads, 25 to 75 parts by weight of the cross-linkable monomer, and 0.5 to 1.3 parts by weight of the photopolymerization initiator based on 100 parts by weight of the vinyl monomer in the microchannel or microchamber; and
exposing the same to UV radiation.

15. A method of concentrating cells or viruses using the microfluidic device according to any one of claims 1 to 14, comprising:
flowing a sample containing the cells or viruses through the microchannel or the microchamber and thereby attaching the cells or viruses to a hydrophobic porous polymer bonded to the wall of the microchannel or microchamber.

16. The method of claim 15, wherein the sample containing the cells or viruses has a pH from pH 2.0 to pH 7.0.

17. A method of disrupting cells or viruses using the microfluidic device according to any one of claims 1 to 14, comprising:
flowing a sample containing cells or viruses through the microchannel or microchamber of the microfluidic device and thereby attaching cells or viruses to a hydrophobic porous polymer bonded to the wall of the microchannel or microchamber; and
exposing the hydrophobic porous polymer in the microchannel or microchamber to light, thereby generating heat disrupting the cells or viruses.

18. A method of amplifying a target DNA from cells or viruses using the microfluidic device according to any one of claims 1 to 14, comprising:
flowing a sample containing cells or viruses through the microchannel or microchamber of the microfluidic device, thereby attaching cells or viruses to a hydrophobic porous polymer bonded to the wall of the microchannel or microchamber;
exposing the hydrophobic porous polymer in the microchannel and the microchamber to light, thereby generating heat disrupting the cells or viruses; and
carrying out PCR using the lysate of the disrupted cells or viruses as a template.

19. The method of claim 18, wherein the PCR is carried out in the PCR reaction means of the microfluidic device.

## Patentansprüche

1. Mikrofluidische Vorrichtung, umfassend wenigstens einen Einlass und wenigstens einen Auslass, welche durch einen Mikrokanal oder eine Mikrokammer miteinander verbunden sind, wobei ein hydrophobes poröses Polymer an die Wand des Mikrokanals oder der Mikrokammer gebunden ist und Magnetbeads an das hydrophobe poröse Polymer gebunden sind, wobei der Wasserkontaktwinkel des hydrophoben porösen Polymers 70° bis 90° beträgt.

2. Vorrichtung nach Anspruch 1, außerdem umfassend eine lichtemittierende Vorrichtung zum Bereitstellen von Licht im Inneren des Mikrokanals oder der Mikrokammer.

3. Vorrichtung nach Anspruch 1 oder 2, außerdem umfassend ein PCR-Reaktionsmittel, umfassend:
eine Reaktionskammer, welche mit dem Mikrokanal oder der Mikrokammer in Fluidverbindung ist; und
ein Mittel zur Temperatureinstellung zum Einstellen der Temperatur in der Reaktionskammer.

4. Vorrichtung nach Anspruch 3, wobei das Mittel zur Temperatureinstellung einen Heizer zum Bereitstellen von Wärme für die Reaktionskammer und einen Kühler zum Kühlen der Reaktionskammer umfasst.

5. Vorrichtung nach einem der Ansprüche 1 bis 4, wobei das hydrophobe poröse Polymer ein Polymer von Vinylmonomeren ist.

6. Vorrichtung nach Anspruch 5, wobei jedes der Vinylmonomere ein Monomer, ausgewählt aus der Gruppe, bestehend aus einem C₁-C₂₀-Alkylacrylat, einem C₁-C₂₀-Alkylmethacrylat und einem Styrol, ist.

7. Vorrichtung nach einem der Ansprüche 1 bis 6, wobei das hydrophobe poröse Polymer ein vernetztes hydrophobes poröses Polymer ist.

8. Vorrichtung nach Anspruch 7, wobei das hydrophobe poröse Polymer durch ein aliphatisches vernetzbares Monomer vernetzt ist.

9. Vorrichtung nach Anspruch 8, wobei das aliphatische vernetzbare Monomer ein Monomer ist, das ausgewählt ist aus der Gruppe bestehend aus einem Ethylenglycoldimethacrylat, einem Ethylenglycoldiacrylat, einem Trimethylolpropandiacrylat, einem Trimethylolpropantriacrylat, einem Trimethylolpropandimethacrylat, einem Trimethylolpropantrimethacrylat, einem Divinylketon, Arylacrylat, Diallylmaleat, Diallylfumarat, Diallylsuccinat, Diallylcarbonat, Diallylmalonat, Diallyloxalat, Diallyladipat, Diallylsebacat, Divinylsebacat, N,N`-Methylendiacrylamid und N,N`-Methylendimethacrylamid.

10. Vorrichtung nach einem der Ansprüche 1 bis 9, wobei das hydrophobe poröse Polymer durch ein aromatisches vernetzbares Monomer vernetzt ist.

11. Vorrichtung nach Anspruch 10, wobei das aromatische vernetzbare Monomer ein Monomer ist, das ausgewählt ist aus der Gruppe bestehend aus einem Divinylbenzol, einem Trivinylbenzol, einem Divinyltoluol, einem Divinylnaphthalin, einem Diallylphthalat, einem Divinylxylol und einem Divinylethylbenzol.

12. Vorrichtung nach einem der Ansprüche 1 bis 11, wobei die Magnetbeads kovalent an das hydrophobe poröse Polymer gebunden sind.

13. Vorrichtung nach einem der Ansprüche 1 bis 12, wobei das hydrophobe poröse Polymer ein Produkt ist, welches erhältlich ist aus einer Photopolymerisation von einem Vinylmonomer, Magnetbeads mit einer Vinylgruppe an ihrer Oberfläche, einem vernetzbaren Monomer mit zwei oder mehr radikalisch polymerisierbaren Doppelbindungen, einem Photopolymerisationsinitiator und einer funktionellen Gruppe mit einer radikalisch polymerisierbaren Vinyl-Doppelbindung, wobei die funktionelle Gruppe in der Wand des Mikrokanals oder der Mikrokammer vorhanden ist.

14. Vorrichtung nach Anspruch 13, wobei die Photopolymerisation durchgeführt wird durch:
Injizieren von 15 bis 33 Gewichtsteilen der Magnetbeads, 25 bis 75 Gewichtsteilen des vernetzbaren Monomers und 0,5 bis 1,3 Gewichtsteilen des Photopolymerisationsinitiators,
bezogen auf 100 Gewichtsteile des Vinylmonomers, in den Mikrokanal oder die Mikrokammer; und
Aussetzen derselben einer UV-Strahlung.

15. Verfahren zum Konzentrieren von Zellen oder Viren unter Verwendung der mikrofluidischen Vorrichtung gemäß einem der Ansprüche 1 bis 14, umfassend:
Leiten einer Probe, welche die Zellen oder Viren enthält, durch den Mikrokanal oder die Mikrokammer und dadurch Anlagern der Zellen oder Viren an einem hydrophoben porösen Polymer, das an die Wand des Mikrokanals oder der Mikrokammer gebunden ist.

16. Verfahren nach Anspruch 15, wobei die Probe, welche die Zellen oder Viren enthält, einen pH von pH 2,0 bis pH 7,0 aufweist.

17. Verfahren zum Aufbrechen von Zellen oder Viren unter Verwendung der mikrofluidischen Vorrichtung gemäß einem der Ansprüche 1 bis 14, umfassend:
Leiten einer Probe, die Zellen oder Viren enthält, durch den Mikrokanal oder die Mikrokammer der mikrofluidischen Vorrichtung und dadurch Anlagern der Zellen oder Viren an einem hydrophoben porösen Polymer, das an die Wand des Mikrokanals oder der Mikrokammer gebunden ist; und
Belichten des hydrophoben porösen Polymers in dem Mikrokanal oder der Mikrokammer, wodurch Wärme erzeugt wird, welche die Zellen oder Viren aufbricht.

18. Verfahren zum Amplifizieren einer Ziel-DNA aus Zellen oder Viren unter Verwendung der mikrofluidischen Vorrichtung gemäß einem der Ansprüche 1 bis 14, umfassend:
Leiten einer Probe, die Zellen oder Viren enthält, durch den Mikrokanal oder die Mikrokammer der mikrofluidischen Vorrichtung, wodurch Zellen oder Viren an einem hydrophoben porösen Polymer angelagert werden, das an die Wand des Mikrokanals oder der Mikrokammer gebunden ist;
Belichten des hydrophoben porösen Polymers in dem Mikrokanal oder der Mikrokammer, wodurch Wärme erzeugt wird, welche die Zellen oder Viren aufbricht; und
Ausführen einer PCR unter Verwendung des Lysats der aufgebrochenen Zellen oder Viren als Matrize.

19. Verfahren nach Anspruch 18, wobei die PCR in dem PCR-Reaktionsmittel der mikrofluidischen Vorrichtung ausgeführt wird.

## Revendications

1. Dispositif microfluidique comprenant au moins un orifice d'entrée et au moins un orifice de sortie qui sont connectés l'un à l'autre à travers un microcanal ou une microchambre, dans lequel un polymère poreux hydrophobe est lié à la paroi du microcanal ou de la microchambre et des billes magnétiques sont liées au polymère poreux hydrophobe, dans lequel l'angle de contact avec l'eau du polymère poreux hydrophobe est de 70° à 90°.

2. Dispositif selon la revendication 1, comprenant en outre un dispositif d'émission de lumière pour fournir de la lumière à l'intérieur du microcanal ou de la microchambre.

3. Dispositif selon la revendication 1 ou 2, comprenant en outre un moyen de réaction PCR comprenant :
une chambre réactionnelle, qui se trouve en communication fluide avec le microcanal ou la microchambre ; et
un moyen d'ajustement de température pour l'ajustement de la température dans la chambre réactionnelle.

4. Dispositif selon la revendication 3, dans lequel le moyen d'ajustement de température comprend un dispositif chauffant pour fournir de la chaleur à la chambre réactionnelle et un dispositif de refroidissement pour refroidir la chambre réactionnelle.

5. Dispositif selon l'une quelconque des revendications 1 à 4, dans lequel le polymère poreux hydrophobe est un polymère de monomères de vinyle.

6. Dispositif selon la revendication 5, dans lequel chacun des monomères de vinyle est un monomère sélectionné dans le groupe constitué d'un acrylate d'alkyle en C₁ à C₂₀, d'un méthacrylate d'alkyle en C₁ à C₂₀, et d'un styrène.

7. Dispositif selon l'une quelconque des revendications 1 à 6, dans lequel le polymère poreux hydrophobe est un polymère poreux hydrophobe réticulé.

8. Dispositif selon la revendication 7, dans lequel le polymère poreux hydrophobe est réticulé par un monomère aliphatique réticulable.

9. Dispositif selon la revendication 8, dans lequel le monomère aliphatique réticulable est un monomère sélectionné dans le groupe constitué d'un diméthacrylate d'éthylène glycol, d'un diacrylate d'éthylène glycol, d'un diacrylate de tri-méthylol propane, d'un triacrylate de tri-méthylol propane, d'un diméthacrylate de tri-méthylol propane, d'un triméthacrylate de tri-méthylol propane, d'une divinylcétone, d'un acrylate d'aryle, d'un maléate de diallyle, d'un fumarate de diallyle, d'un succinate de diallyle, d'un carbonate de diallyle, d'un malonate de diallyle, d'un oxalate de diallyle, d'un adipate de diallyle, d'un sébaçate de diallyle, d'un sébaçate de divinyle, d'un N,N'-méthylène diacrylamide, et d'un N,N'-méthylène diméthacrylamide.

10. Dispositif selon l'une quelconque des revendications 1 à 9, dans lequel le polymère poreux hydrophobe est réticulé par un monomère réticulable aromatique.

11. Dispositif selon la revendication 10, dans lequel le monomère réticulable aromatique est un monomère sélectionné dans le groupe constitué d'un divinylbenzène, d'un trivinylbenzène, d'un divinyltoluène, d'un divinylnaphtalène, d'un phtalate de diallyle, d'un divinylxylène, et d'un divinyléthylbenzène.

12. Dispositif selon l'une quelconque des revendications 1 à 11, dans lequel les billes magnétiques sont liées de manière covalente au polymère poreux hydrophobe.

13. Dispositif selon l'une quelconque des revendications 1 à 12, dans lequel le polymère poreux hydrophobe est un produit qui peut être obtenu à partir d'une photopolymérisation d'un monomère de vinyle, de billes magnétiques ayant un groupe vinyle à leur surface, d'un monomère réticulable ayant deux ou plusieurs doubles liaisons polymérisables par radicaux libres, d'un initiateur de photopolymérisation, et d'un groupe fonctionnel ayant une double liaison vinylique polymérisable par radicaux libres, ledit groupe fonctionnel existant dans la paroi du microcanal ou de la microchambre.

14. Dispositif selon la revendication 13, dans lequel la photopolymérisation est conduite par :
injection de 15 à 33 parties en poids de billes magnétiques, de 25 à 75 parties en poids du monomère réticulable, et de 0,5 à 1,3 partie en poids de l'initiateur de photopolymérisation sur la base de 100 parties en poids du monomère de vinyle dans le microcanal ou la microchambre ; et
son exposition aux rayonnements UV.

15. Procédé de concentration de cellules ou de virus utilisant le dispositif microfluidique selon l'une quelconque des revendications 1 à 14, comprenant :
l'écoulement d'un échantillon contenant les cellules ou les virus à travers le microcanal ou la microchambre et la fixation de là des cellules ou des virus à un polymère poreux hydrophobe lié à la paroi du microcanal ou de la microchambre.

16. Procédé selon la revendication 15, dans lequel l'échantillon contenant les cellules ou les virus présente un pH allant du pH 2,0 au pH 7,0.

17. Procédé de rupture de cellules ou de virus utilisant le dispositif microfluidique selon l'une quelconque des revendications 1 à 14, comprenant :
l'écoulement d'un échantillon contenant les cellules ou les virus à travers le microcanal ou la microchambre du dispositif microfluidique et de là la fixation des cellules ou des virus à un polymère poreux hydrophobe lié à la paroi du microcanal ou de la microchambre ; et
l'exposition du polymère poreux hydrophobe dans le microcanal ou la microchambre à de la lumière, générant de là de la chaleur brisant les cellules ou les virus.

18. Procédé d'amplification d'un ADN cible à partir de cellules ou de virus utilisant le dispositif microfluidique selon l'une quelconque des revendications 1 à 14, comprenant :
l'écoulement d'un échantillon contenant les cellules ou les virus à travers le microcanal ou la microchambre du dispositif microfluidique, fixant de là les cellules ou les virus à un polymère poreux hydrophobe lié à la paroi du microcanal ou de la microchambre ;
l'exposition du polymère poreux hydrophobe dans le microcanal et la microchambre à de la lumière, générant de là de la chaleur brisant les cellules ou les virus ; et
la conduite d'une PCR en utilisant le lysat des cellules ou des virus brisés-es comme matrice.

19. Procédé selon la revendication 18, dans lequel la PCR est conduite dans le moyen réactionnel de PCR du dispositif microfluidique.
